# EUROPEAN PATENT SPECIFICATION

(11) **EP 3 015 113 B1**
(45) Date of publication and mention of the grant of the patent: **28.08.2019**
(21) Application number: 15192456.0
(22) Date of filing: 30.10.2015
(51) Int. Cl.: A61K 38/12, A61P 41/00

(54) **TRANSPLANT SITE-FORMING AGENT, TRANSPLANT SITE-FORMING DEVICE, ANGIOGENIC AGENT AND ANGIOGENIC DEVICE**
TRANSPLANTATIONSORTBILDUNGSMITTEL, TRANSPLANTATIONSORTBILDUNGSVORRICHTUNG, ANGIOGENES MITTEL UND ANGIOGENE VORRICHTUNG
AGENT DE FORMATION DE SITE DE TRANSPLANTATION, DISPOSITIF DE FORMATION DE SITE DE TRANSPLANTATION, AGENT ANGIOGÉNIQUE ET DISPOSITIF ANGIOGÉNIQUE

(30) Priority: 31.10.2014 JP 2014223174; 27.10.2015 JP 2015211066
(43) Date of publication of application: 04.05.2016
(73) Proprietor: Kyoto University, Kyoto-shi, Kyoto 606-8501 (JP); ARKRAY, Inc., Minami-ku Kyoto-shi Kyoto 601-8045 (JP)
(72) Inventor: IWATA, Hiroo, Kyoto-shi, Kyoto 606-8501 (JP); KUWABARA, Rei, Kyoto-shi, Kyoto 606-8501 (JP); HIRANO, Kunio, Kyoto-shi, Kyoto 602-0008 (JP)
(74) Representative: Dehns

(56) References cited:
- ABE Y ET AL: "Wound healing acceleration of a novel transforming growth factor-beta inducer, SEK-1005", EUROPEAN JOURNAL OF PHARMACOLOGY, ELSEVIER SCIENCE, NL, vol. 408, no. 2, 1 January 2000 (2000-01-01), pages 213-218, XP002964357, ISSN: 0014-2999, DOI: 10.1016/S0014-2999(00)00766-4
- SANO HIROKI ET AL: "Coils coated with the cyclic peptide SEK-1005 accelerate intra-aneurysmal organization", NEUROSURGERY : OFFICIAL JOURNAL OF THE CONGRESS OF NEUROLOGICAL SURGEONS, LIPPINCOTT WILLIAMS & WILKINS, US, vol. 67, no. 4, 1 October 2010 (2010-10-01), pages 984-992, XP009188472, ISSN: 1524-4040
- N. M. LUAN ET AL: "Long-Term Allogeneic Islet Graft Survival in Prevascularized Subcutaneous Sites Without Immunosuppressive Treatment", AMERICAN JOURNAL OF TRANSPLANTATION, vol. 14, no. 7, 6 July 2014 (2014-07-06), pages 1533-1542, XP055248821, DK ISSN: 1600-6135, DOI: 10.1111/ajt.12739

## Description

### Technical Field

The invention relates to a transplant site-forming agent or a transplant site-forming device for use in forming a transplant site, for inducing angiogenesis, for transplanting a transplant material, for promoting engraftment of a transplant material at a transplant site, for treating diabetes, for controlling a blood glucose level, and for controlling a plasma insulin concentration.

The invention further relates to an angiogenic agent or an angiogenic device for use in inducing angiogenesis and for use in inducing angiogenesis that is suitable for obtaining effects of: forming a transplant site; transplanting a transplant material; promoting engraftment of a transplant material at a transplant site; treating diabetes; controlling a blood glucose level; and controlling a plasma insulin concentration (hereinafter, these effects are also referred to as "forming a transplant site" or "formation of a transplant site").

### Background Art

As a method of treating diabetes, there is a method in which islets of Langerhans (pancreatic islets), which function to control a level of glucose in blood by secreting insulin, are transplanted in a patient body. In this method, although a tissue of pancreatic islets is generally transplanted in the liver, a study has been made on a method in which pancreatic islets are transplanted under the skin, as a method that is less invasive and easier to remove the transplanted pancreatic islets upon occurrence of abnormal changes. In the specification, a graft of islets of Langerhans, which has an ability of producing insulin as a transplant material, may be simply referred to as pancreatic islets.

In a case in which a tissue such as pancreatic islets is transplanted under the skin, there is a problem in that the engraftment rate of the transplanted tissue is low because of insufficiency in oxygen and nutrition in a subcutaneous tissue, in which blood vessels having a small diameter are not sufficiently developed. As a means for solving this problem, a device which promotes formation of blood vessels having a small diameter in a tissue around a site under the skin at which it is implanted, has been proposed (see, for example, Japanese Patent No. 3089299).

In addition, there is a report that generation of blood vessels having a small diameter was observed at a site under the skin of a diabetic rat at which a device containing basic fibroblast growth factors (bFGF), as a substance for inducing angiogenesis, was implanted, and that the blood glucose level was normalized during a long period of time after transplanting pancreatic islets at the site after removing the device therefrom (see, for example, American Journal of Transplantation, Volume 14, Issue 7, pp.1533-1542, July 2014).

Seeking a wide variety of substances that are suitable for forming a transplant site that exhibits an excellent engraftment rate of a transplant material after being transplanted, an excellent ability of inducing angiogenesis, and an excellent ability of inducing angiogenesis that is suitable for formation of a transplant site, would benefit development of various methods of transplantation. Further, if production of a transplant material from induced pluripotent stem cells (such as iPS cells) or embryonic stem cells (such as ES cells) is put into practice in future, it is considered desirable that the transplant material can be readily removed from the transplant site, in case that any troubles may occur. Accordingly, it is expected that the importance of transplantation of a transplant material under the skin as an option of the treatment method will increase, and so will the importance of finding various substances suitable for this purpose.

In view of the circumstances set forth above, the invention provides a transplant site-forming agent and a transplant site-forming device that exhibit an excellent engraftment rate of a transplant material, an angiogenic agent and an angiogenic device that exhibit an excellent ability of inducing angiogenesis, and an angiogenic agent and an angiogenic device that exhibit an excellent ability of inducing angiogenesis that is suitable for formation of a transplant material.

### Summary of the Invention

The Embodiments of the invention include the following.
<1> SEK-1005 (structural formula: C₄₅H₇₀N₈O₁₃ : name; Ser,3-hydroxy-N-[2-hydroxy-1-oxo-2-tetrahydro-2-hydroxy-6-methyl-5-(2-methylpropyl)-2H-pyran-2-yl-propyl]-Leu-Pip(hexahydro-3-pyridazinecarbonyl)-N-hydroxy-Ala-N-methyl-Phe-Pip-rho-lactone) or a salt thereof for use in a method of forming a transplant site which is suitable for transplantation of a transplant material, said method comprising contacting SEK-1005 or a salt thereof with a body tissue in a living body .
<2> The SEK-1005 or salt thereof for use according to <1> for forming a transplant site in a subcutaneous tissue.
<3> The SEK-1005 or salt thereof for use according to <1> or <2>, wherein a transplant material is transplanted to the transplant site, the transplant material being selected from the group consisting of a cell, a tissue, a cell that is formed by differentiation induction of an embryonic stem cell or an induced pluripotent stem cell, and a tissue that is formed by differentiation induction of an embryonic stem cell or an induced pluripotent stem cell.
<4> The SEK-1005 or salt thereof for use according to <1> to <3>, wherein a transplant material that secretes a physiologically active substance is transplanted to the transplant site.
<5> The SEK-1005 or salt thereof for use according to <4>, wherein the transplant material that secretes a physiologically active substance is a tissue or a cell, the tissue or the cell secreting at least one kind of physiologically active substance selected from the group consisting of a hormone, a cytokine, a neurotransmitter and an enzyme.
<6> The SEK-1005 or salt thereof for use according to <4>, wherein the transplant material that secretes a physiologically active substance is a pancreatic islet, a hepatic cell, an adrenal gland, a parathyroid, a cell that produces erythropoietin (such as an interstitial cell in renal tubule), a cell that produces growth hormone (such as a GH-secreting cell in anterior pituitary), a transplant material produced from a somatic stem cell, a transplant material produced from an induced pluripotent stem cell or a transplant material produced from an embryonic stem cell.
<7> The SEK-1005 or salt thereof for use according to any one of from <4> to <6>, wherein the transplant material that secretes a physiologically active substance is a pancreatic islet.
<8> The SEK-1005 or salt thereof for use according to <7>, wherein a plasma insulin concentration of an individual after transplantation of the pancreatic islet becomes higher than a plasma insulin concentration of the individual before the transplantation.
<9> The SEK-1005 or salt thereof for use according to <8>, wherein the plasma insulin concentration of the individual after transplantation of the pancreatic islet is at least twice the plasma insulin concentration of the individual before the transplantation.
<10> The SEK-1005 or salt thereof for use according to <8> or <9>, wherein a state in which the plasma insulin concentration of the individual after transplantation of the pancreatic islet is higher than the plasma insulin concentration of the individual before the transplantation continues for 10 days or more.
<11> The SEK-1005 or salt thereof for use according to any one of from <7> to <10>, wherein a blood glucose level of the individual after transplantation of the pancreatic islet becomes lower than a blood glucose level of the individual before the transplantation.
<12> The SEK-1005 or salt thereof for use according to <11>, wherein a state in which the blood glucose level of the individual after transplantation of the pancreatic islet is lower than the blood glucose level of the individual before the transplantation continues for 10 days or more.
<13> The SEK-1005 or salt thereof for use according to any one of from <1> to <12>, wherein the transplant site is formed by inducing angiogenesis in a subcutaneous tissue.
<14> A transplant site-forming device that comprises the SEK-1005 or salt thereof according to <1>, and a substrate that retains the SEK-1005 or salt thereof for use in forming a transplant site by implanting the transplant site-forming device under the skin of an individual and allowing SEK-1005 or a salt thereof to be released to a tissue surrounding the transplant site-forming device.
<15> The transplant site-forming device for use according to <14>, wherein the substrate is formed from at least one selected from the group consisting of a hydrogel, a dried hydrogel, a sponge, a porous polymer block, a porous body, a porous sheet and a porous film.
<16> The transplant site-forming device for use according to <14> or <15>, wherein the substrate is formed from a biocompatible material.
<17> The transplant site-forming device for use according to any one of from <14> to <16>, that is implanted under a skin of an individual and releases the SEK-1005 or salt thereof to induce angiogenesis in a tissue surrounding the transplant site-forming device.
<18> The transplant site-forming device for use according to <17> that is removed, or is not removed, after angiogenesis has been induced.
<19> SEK-1005 or a salt thereof for use in induction of angiogenesis.
<20> The SEK-1005 or salt thereof according to <19> for use in inducing angiogenesis in a subcutaneous tissue.
<21> The SEK-1005 or salt thereof for use according to <20>, wherein an amount of hemoglobin in the subcutaneous tissue becomes at least twice by the induction of angiogenesis in the subcutaneous tissue.
<22> The SEK-1005 or salt thereof for use according to <21>, wherein a period in which the amount of hemoglobin in the subcutaneous tissue becomes at least twice is from 5 days to 20 days.
<23> The SEK-1005 or salt thereof for use according to any one of from <20> to <22>, wherein the subcutaneous tissue is a transplant site for transplanting a transplanting material.
<24> The SEK-1005 or salt thereof for use according to <23>, wherein the transplant site is for transplanting a transplant material that secretes a physiologically active substance.
<25> The SEK-1005 or salt thereof for use according to <24>, wherein the transplant material that secretes a physiologically active substance is a tissue or a cell, the tissue or the cell secreting at least one kind of physiologically active substance selected from the group consisting of a hormone, a cytokine, a neurotransmitter and an enzyme.
<26> The SEK-1005 or salt thereof for use according to <24>, wherein the transplant material that secretes a physiologically active substance is a pancreatic islet, a hepatic cell, an adrenal gland, a parathyroid, a cell that produces erythropoietin (such as an interstitial cell in renal tubule), a cell that produces growth hormone (such as a GH-secreting cell in anterior pituitary), a transplant material produced from a somatic stem cell, a transplant material produced from an induced pluripotent stem cell or a transplant material produced from an embryonic stem cell.
<27> The SEK-1005 or salt thereof for use according to any one of from <24> to <26>, wherein the transplant material that secretes a physiologically active substance is a pancreatic islet.
<28> The SEK-1005 or salt thereof for use according to <27>, wherein a plasma insulin concentration of an individual after transplantation of the pancreatic islet becomes higher than a plasma insulin concentration of the individual before the transplantation.
<29> The SEK-1005 or salt thereof for use according to <28>, wherein the plasma insulin concentration of the individual after transplantation of the pancreatic islet is at least twice the plasma insulin concentration of the individual before the transplantation.
<30> The SEK-1005 or salt thereof for use according to <28> or <29>, wherein a state in which the plasma insulin concentration of the individual after transplantation of the pancreatic islet is higher than the plasma insulin concentration of the individual before the transplantation continues for 10 days or more.
<31> The SEK-1005 or salt thereof for use according to any one of from <27> to <30>, wherein a blood glucose level of the individual after transplantation of the pancreatic islet is lower than a blood glucose level of the individual before the transplantation.
<32> The SEK-1005 or salt thereof for use according to <31>, wherein a state in which the blood glucose level of the individual after transplantation of the pancreatic islet is lower than the blood glucose level of the individual before the transplantation continues for 10 days or more.
<33> The SEK-1005 or salt thereof for use according to any one of from <19> to <32> that is suitable for forming a transplant site.
<34> An angiogenic device that comprises the SEK-1005 or salt thereof according to <1> and a substrate that retains the SEK-1005 or salt thereof for use in induction of angiogenesis.
<35> The angiogenic device for use according to <34>, wherein the substrate is formed from at least one selected from the group consisting of a hydrogel, a dried hydrogel, a sponge, a porous polymer block, a porous body, a porous sheet and a porous film.
<36> The angiogenic device for use according to <34> or <35>, wherein the substrate is formed from a biocompatible material.
<37> The angiogenic device for use according to any one of from <34> to <36>, that is implanted under a skin of an individual and releases the SEK-1005 or salt thereof to induce angiogenesis in a surrounding tissue.
<38> The angiogenic device for use according to <37> that is removed, or is not removed, after the induction of angiogenesis.
<39> The SEK-1005 or salt thereof for use according to <19> or angiogenic device for use according to <34> for inducing angiogenesis that is suitable for obtaining effects of: forming a transplant site; transplanting a transplant material; promoting engraftment of a transplant material at a transplant site; treating diabetes; controlling a blood glucose level; and controlling a plasma insulin concentration.

According to the embodiments, it is possible to provide a transplant site-forming agent and a transplant site-forming device that exhibit an excellent engraftment rate of a transplant material; an angiogenic agent and an angiogenic device that exhibit an excellent ability of inducing angiogenesis; and an angiogenic agent and an angiogenic device that exhibit an excellent ability of inducing angiogenesis that is suitable for forming a transplant site, which exhibits an excellent engraftment rate of a transplant material.

### Brief Explanation of the Drawings

Fig. 1 is a photograph of a surrounding tissue after removing an agarose rod that has been implanted under the skin of the back of a diabetic rat.
Fig. 2 is a photograph of a slice of a subcutaneous tissue stained with hematoxylin-eosin (HE) at a contact portion with the agarose rod.
Fig. 3 is a graph showing measurement results of the amount of hemoglobin in a subcutaneous tissue at a contact portion with the agarose rod.
Fig. 4 is a graph showing measurement results of blood glucose levels of recipients after being transplanted with pancreatic islets at a site from which an agarose rod containing SEK-1005 had been removed.
Fig. 5 is a graph showing measurement results of blood glucose levels of recipients after being transplanted with pancreatic islets at a site from which an agarose rod not containing SEK-1005 had been removed.
Fig. 6 is a graph showing measurement results of plasma insulin concentrations of recipients after being transplanted with pancreatic islets.
Fig. 7 is a graph showing the results of a glucose tolerance test of recipients at 50 to 55 days or at 100 to 105 days after the transplantation.
Fig. 8 is a HE-stained image and an immunofluorescent-stained image of a tissue slice of a transplant site at 106 days after the transplantation of pancreatic islets.

### Detailed description

In the following, the embodiments are described in detail. However, the following description does not limit the scope of the invention.

The numerical range indicated by "from A to B" refers to a range including A and B as minimum and maximum values.

The term "angiogenic" or "inducing angiogenesis" refers to an ability of inducing angiogenesis or a function of inducing angiogenesis in a body tissue. More specifically, the term refers to increasing the amount of blood vessels by contacting the angiogenic agent or the angiogenic device with a body tissue, compared to the amount of blood vessels in a case in which the body tissue is not contacted with the angiogenic agent or the angiogenic device.

The term "treating" includes suppressing worsening of a symptom, alleviating a symptom, and treating or preventing a complication, in addition to allowing a symptom to disappear.

The term "transplant site" refers to a site of a body tissue to which a transplant material is to be transplanted.

### <Transplant site-forming agent>

The transplant site-forming agent includes SEK-1005 or a salt thereof. SEK-1005, i.e., Ser,3-hydroxy-N-[2-hydroxy-1-oxo-2-tetrahydro-2-hydroxy-6-methyl-5-(2-methylpropyl)-2H-pyran-2-yl-propyl]-Leu-Pip(hexahydro-3-pyridazinecarbonyl)-N-hydroxy-Ala-N-methyl-Phe-Pip-rho-lactone, is a compound represented by the following structural formula. SEK-1005 is a cyclic peptide that can be isolated from a culture solution or its dried product of Actinomycetes Streptomyces nobilis. With the transplant site-forming agent, a transplant site that exhibits an excellent engraftment rate of a transplanted tissue can be formed.

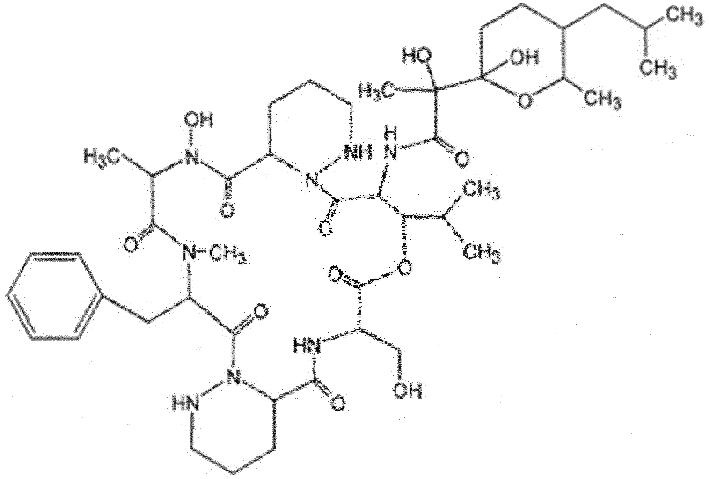

Streptomyces nobilis, from which SEK-1005 can be obtained, is available from public institutions. For example, Streptomyces nobilis is preserved in RIKEN, Japan, as JCM4274; in the United States as ATCC19252; and in the Netherlands as CBS198.65.

The method of obtaining SEK-1005 is not particularly limited. For example, SEK-1005 may be obtained from Streptomyces nobilis or other natural substances, or may be synthesized by a bioengineering method or an organic chemical method. A specific method of obtaining SEK-1005 from Streptomyces nobilis is described in, for example, WO 96/12732.

Various reports have been made with regard to pharmacological functions or application as chemicals of SEK-1005. For example, Japanese Patent Application Laid-Open (JP-A) No. H10-259134 describes a wound curing promoter that includes SEK-1005.

However, no report has been made with regard to utilizing SEK-1005 for forming a transplant site, for inducing angiogenesis or for inducing angiogenesis that is suitable for forming a transplant site, until the inventors find the usefulness of SEK-1005 for these purposes.

The reason why the transplant site formed with the transplant site-forming agent exhibits an excellent engraftment rate is not clear, but is presumably as follows. The inventors consider that when a body tissue is contacted with SEK-1005 included in the transplant site-forming agent, generation of blood vessels is induced in the body tissue. The inventors consider the induced generation of blood vessels is suitable for engraftment of a transplant material and, as a result, a transplant site that is necessary for engraftment of the transplant material can be formed.

The site in the body tissue to which the transplant site-forming agent is to be contacted is not particularly restricted, and examples of the site include a subcutaneous tissue. In a case in which the transplant site-forming agent is contacted with a subcutaneous tissue, the portion of the subcutaneous tissue is not particularly limited, and may be at a surface (an interface with a dermic layer) or inside the same.

The amount of increase of blood vessels generated by the transplant site-forming agent can be determined by, for example, comparing the amount of hemoglobin in the body tissue before and after contacting the transplant site-forming agent. Although it depends on the content of SEK-1005, the position of the body tissue, or the contact method, the transplant site-forming agent can increase the amount of hemoglobin, in a case of a subcutaneous tissue, to at least twice, at least three times, or at least four times the amount of hemoglobin before contacting the transplant site-forming agent to the subcutaneous tissue.

The amount of the transplant site-forming agent suitable for forming a transplant site that exhibits an excellent engraftment rate of a transplanted tissue is not particularly limited. For example, the amount of the transplant site-forming agent is preferably an amount that is enough for inducing angiogenesis in terms of the amount of hemoglobin of from 2 mg/g tissue to 50 mg/g tissue, more preferably from 3 mg/g tissue to 40 mg/g tissue, further preferably from 4 mg/g tissue to 30 mg/g tissue, particularly preferably from 5 mg/g tissue to 25 mg/g tissue.

The amount of compounding SEK-1005 or the transplant site-forming agent in the transplant site-forming device is not particularly limited. For example, it is preferably an amount that is enough for increasing the amount of hemoglobin to 5 times to 150 times, more preferably to from 10 times to 100 times, further preferably to from 15 times to 70 times the amount of hemoglobin before implanting the transplant site-forming device to a subcutaneous tissue for 10 days.

The amount of compounding SEK-1005 or the transplant site-forming agent depends on various factors and is not particularly limited. For example, it is preferably an amount that is enough for increasing the amount of hemoglobin, by implanting the transplant site-forming device in a subcutaneous tissue for 10 days, to from 2 times to 15 times, more preferably from 2 times to 10 times, further preferably from 2 times to 5 times the amount of hemoglobin in a case in which a device not including SEK-1005 or the transplant site-forming agent is implanted in the subcutaneous tissue under the same conditions.

The method of contacting the transplant site-forming agent with a body tissue is not particularly limited, and examples thereof include a method of implanting in a body with other material(s) such that the transplant site-forming agent is released, and a method of administering by injection on a regular basis. From the viewpoint of securing the time for the transplant site-forming agent to contact a body tissue, a method of implanting with other material(s) such that the transplant site-forming agent is released is preferred. This method is preferred also because a space for transplanting a transplant material is created after removing the transplant site-forming agent. The size (area) of the body tissue to be contacted with the transplant site-forming agent is not particularly limited, and may be selected according to the treatment method.

The number of times for contacting the transplant site-forming agent with a body tissue is not particularly limited. For example, a desired transplant site may be formed through a single contact or through multiple contacts. The time for contacting the transplant site-forming agent with a body tissue is not particularly limited. Although it depends on the position of the body tissue, the amount of the transplant site-forming agent or the method of contacting with the body tissue, the time period for contacting the transplant site-forming agent with a body tissue is approximately from 5 days to 20 days, for example. In an embodiment, in a case of contacting the transplant site-forming agent to a subcutaneous tissue, the time period in which the amount of hemoglobin in the subcutaneous tissue becomes at least twice is from 5 days to 20 days after the contact.

The transplant site-forming agent may consist only of SEK-1005, or may include a component other than SEK-1005. Examples of the component include a diluent, a disintegrant, a binder, a lubricant, a surfactant, a solubilizer, a stabilizer, a tonicity agent, a suspending agent, an emulsifier, a buffer, and a solvent. When the transplant site-forming agent includes a component other than SEK-1005, the content of SEK-1005 is not particularly limited, as long as SEK-1005 exhibits its effect as an active ingredient of the transplant site-forming agent.

The transplant site that is formed by the transplant site-forming agent can be used for transplantation of various kinds of transplant materials. In particular, the transplant site is suitable for transplantation of a transplant material that functions irrespective of the position for the transplantation, and examples of such transplant materials include a tissue or a cell that secretes a physiologically active substance such as a hormone, a cytokine, a neurotransmitter and an enzyme. Specific examples thereof include a pancreatic islet, a hepatic cell, an adrenal gland, a parathyroid, a cell that produces erythropoietin (such as an interstitial cell in renal tubule), and a cell that produces a growth hormone (such as a GH-secreting cell in anterior pituitary). Further, the transplant site-forming agent can be used for transplantation of a transplant material produced by inducing differentiation of a somatic stem cell, an induced pluripotent stem cell (iPS cell) or an embryonic stem cell (ES cell).

Examples of the transplant material to be transplanted to a transplant site formed with the transplant site-forming agent include a tissue (including an organ) and a cell that can survive in the transplant site, and the transplant material may be derived from a living body or may be an artificial product. The transplant material may be not only a cell or a tissue of the same kind or a microtissue or a cell of the same kind, but also a cell or a tissue of a different kind or a microtissue or a cell of a different kind. When using a cell or a tissue of the same kind, the cell or the tissue may be not only a cell or a tissue of the same kind and the same system or a microtissue or a cell of the same kind and the same system, but also a cell or a tissue of the same kind but a different system or a microtissue or a cell of the same kind but a different system. The term "same kind" refers to a kind of the transplant material that is the same, the term "different kind" refers to a kind of the transplant material that is different, the term "same kind and same system" refers to a kind that is the same and the genetic composition is common, and the term "same kind but different system" refers to a kind that is the same but the genetic composition is not common.

Transplanting a tissue to a transplant site formed with the transplant site-forming agent is effective in treating various kinds of diseases. It is especially effective for easing symptoms or improving life qualities of a patient of insulin-dependent diabetes, requiring constant control of secretion of insulin in response to blood glucose levels, as compared to other endocrine diseases. Accordingly, the transplant site-forming agent is useful especially in a case of forming a transplant site for transplanting pancreatic islands for treating insulin-dependent diabetes.

Although Type 1 diabetes is a major case of insulin-dependent diabetes, the transplant site-forming agent is effective also for treating type 2 diabetes. As type 2 diabetes becomes advanced, the volume of pancreatic beta cells is decreased and administration of insulin becomes necessary for the treatment. In that case, the transplant site-forming agent can be used for the treatment. Considering that type 2 diabetes becomes advanced as the body becomes more resistant against insulin relative to the ability of the pancreas to produce insulin, conducting the transplantation for preventive purposes, in advance of reaching a stage of requiring insulin administration, may also be effective.

Further, according to the transplant site-forming agent, it is possible to form a transplant site to which a tissue of a different individual can be transplanted.

In an embodiment, the transplant site-forming agent is used for forming a transplant site for transplanting pancreatic islets in a subcutaneous tissue. In the subcutaneous tissue formed with the transplant site-forming agent, blood vessels having a small diameter are formed in an amount sufficient for the engraftment of the pancreatic islets. In a transplant site formed with the transplant site-forming agent, pancreatic islets can secrete a sufficient amount of insulin for controlling a blood glucose level, and can increase a plasma insulin concentration of an individual after the transplantation from the level of the same before the transplantation. As a result, it is possible to control the blood glucose level of an individual after the transplantation to be lower than that before the transplantation. For example, it is possible to increase the plasma insulin concentration of an individual after transplanting pancreatic islets to at least twice, at least three times, or at least four times the plasma insulin concentration before the transplantation. Further, for example, it is possible to control the blood glucose level of an individual after transplanting pancreatic islets to be close to a normal level, or to control the fluctuation of the blood glucose level to be close to a normal state.

In an embodiment, pancreatic islets that have been transplanted to a transplant site formed with the transplant site-forming agent can secrete a sufficient amount of insulin for enabling continuous control of a blood glucose level. For example, it is possible to continue a state in which the plasma insulin concentration of an individual after transplanting the pancreatic islets continues to be higher than that before the transplantation, or a state in which the blood glucose level of an individual after transplanting pancreatic islets continues to be lower than that before the transplantation, for 10 days or more, 20 days or more, 30 days or more, 60 days or more, 90 days or more, 100 days or more, 110 days or more, or 120 days or more.

In an embodiment, a transplant material is engrafted to a transplant site formed with the transplant site-forming agent. The reason for this is considered to be that SEK-1005 included in the transplant site-forming agent functions to form a suitable transplant site. The term "engraft" refers to a state in which a transplanted material after being transplanted exerts a desired function, and the same applies in the following.

### <Transplant site-forming device>

The transplant site-forming device includes SEK-1005 or the transplant site-forming agent and a substrate that retains SEK-1005 or the transplant site-forming agent.

When a body tissue is contacted with the transplant site-forming device, the transplant site-forming agent is released from inside the transplant site-forming device that is in contact with the body tissue, and the transplant site-forming agent acts on the body tissue to form a transplant site that exhibits a high engraftment rate of a transplant material after the transplantation. Details of the transplant site-forming agent included in the transplant site-forming device are as described above.

The substrate included in the transplant site-forming device may be formed from a biocompatible material having a shape of various kinds, such as a stick, a plate, a sheet, a tube, a rod and a liquid. The biocompatible material is not particularly limited, as long as it can release the transplant site-forming agent from the transplant site-forming device, and examples thereof include organic materials such as natural polymers and synthetic polymers, and inorganic materials such as metals, metal oxides and ceramics.

Examples of the natural polymers include a hydrogel, a dried hydrogel, a sponge, a porous polymer block, a porous body, a porous sheet and a porous film. Examples of the material for the substrate include cellulose derivatives such as agarose, agar, cellulose and various cellulose derivatives such as hydroxypropyl methylcellulose (HPMC), sodium carboxymethyl cellulose (CMC-Na) and hydroxyethyl cellulose (HEC); polysaccharides and derivatives thereof, such as alginic acid, starch, dextran, pullulan, pectin, hyaluronic acid, chondroitin sulfate, heparin, chitin and chitosan; and proteins and protein derivatives such as gelatin, albumin, collagen and fibrin.

Examples of the synthetic polymers include polyvinyl alcohol, polyvinyl pyrrolidone, polyethylene glycol, poly-2-hydroxyethyl methacrylate, poly-2-hydroxyethyl acrylate, polyacrylamide, polyisopropyl acrylamide, polyacrylic acid, polymethacrylic acid, polysulfone, polyethylene, polypropylene, polycarbonate, polytetrafluloroethylene, polyethylene terephtalate, and copolymers formed from monomers of these polymers. Other examples include a polyion complex of a polyanion and a polycation, such as poly-L-lysine.

Examples of the inorganic materials include stainless steel, titanium, titanium alloy, gold, gold alloy, platinum, platinum alloy, alumina, zirconia, apatite hydroxide, calcium carbonate and calcium phosphate.

These materials may be used alone or in combination of two or more kinds. The substrate is preferably formed of a biocompatible material.

The structure of the transplant site-forming device is not particularly limited, as long as the transplant site-forming agent can be released from the inside of the transplant site-forming device. Examples of the structure include a structure in which the transplant site-forming agent is applied by way of impregnating, or coating, to a substrate formed from a hydrogel, a dried hydrogel, a sponge, a porous polymer block, a porous body, a porous sheet, or a porous film ; or a structure in which the transplant site-forming agent is enclosed in a substrate formed from the aforementioned material in the form of a bag, or a tube. The structure of the transplant site-forming device can be selected in consideration of a releasing rate of the transplant site-forming agent from the transplant site-forming device, or a position of a body tissue at which the transplant site-forming device is to be used.

The size or the shape of the transplant site-forming device is not particularly limited, and may be selected depending on the method of using the transplant site-forming device or the position at which the transplant site-forming device is to be used. The transplant site-forming device may be removed after the device has been introduced in a living body and angiogenesis has been induced, or may not be removed. In a case in which the body tissue in which angiogenesis has been induced is used as a transplant site for a transplant material, and the transplant site-forming device is removed after angiogenesis has been induced, the transplant material may be placed in a space created after the removal of the transplant site-forming device. In that case, it is preferred to select the size or the shape of the transplant site-forming device in consideration of the type or the amount of the tissue to be transplanted. In a case in which the transplant site-forming device is not removed after the device has been introduced in a living body and the transplant site has been formed, the transplant site-forming device may have a structure that can accommodate a transplant material therein, and can allow blood vessels that have grown in the surrounding tissue to extend thereto, or cannot. The transplant site-forming device may have structure in which the substrate is formed of a material decomposable in a body, and disappears entirely or partially after the device has been introduced in a living body and angiogenesis has been induced.

The method of producing the transplant site-forming device is not particularly limited, as long as it is a method by which the transplant site-forming device having a structure that enables the transplant site-forming agent to be released from the inside thereof can be formed.

### <Angiogenic agent>

The angiogenic agent includes SEK-1005 or a salt thereof. When a body tissue is contacted with the angiogenic agent, angiogenesis is induced in the body tissue. With regard to physiologically active functions of SEK-1005, for example, JP-A H10-259134 describes that SEK-1005 functions as a stimulant that activates white blood cells or bone-marrow cells, thereby increasing the amount of a granulation tissue. However, JP-A H10-259134 does not teach or suggest that SEK-1005 functions to induce angiogenesis, or does not provide data concerning generation or regeneration of blood vessels. The function of SEK-1005 of inducing angiogenesis and the usefulness of SEK-1005 as an angiogenic agent were found by the present inventors for the first time.

Further, the present inventors have found that the amount of blood vessels that have been newly generated in a body tissue as a result of contacting with SEK-1005 is much greater than the amount of blood vessels that exist in the body tissue under normal circumstances. This function of SEK-1005 is useful for artificially forming blood vessels of a desired amount in a body tissue in which blood vessels are scarce under normal circumstances, for example.

Also, the inventors have found that a site including blood vessels formed in a body tissue by contacting with SEK-1005 is highly suitable for transplantation of a tissue.

The findings regarding a function of SEK-1005 of inducing angiogenesis as mentioned above have not been revealed. Accordingly, it was difficult to arrive at an idea of utilizing an angiogenic agent including SEK-1005 for the purpose of forming a transplant site, without the knowledge found by the inventors.

The portion of a body tissue to which the angiogenic agent is to be contacted is not particularly limited. The angiogenic agent can induce angiogenesis in an artificial manner, even at a portion with a small amount of blood vessels or substantially without blood vessels. Therefore, the effect of the embodiment is particularly significant when a body tissue at which the amount of blood vessels is relatively small is contacted with the angiogenic agent. Examples of such tissues include a subcutaneous tissue. The portion of a subcutaneous tissue to be contacted with the angiogenic agent is not particularly limited, and may be at a surface of the subcutaneous tissue (an interface with a dermic layer) or inside the same.

Examples of the method of determining the amount of increase of blood vessels formed with the angiogenic agent include a method of comparing the amount of hemoglobin in a body tissue before and after contacting the angiogenic agent. Although it depends on the content of SEK-1005, portion of a body tissue, or method of contacting the angiogenic agent with the body tissue, the angiogenic agent can increase the amount of hemoglobin to at least twice, or at least three times, or at least four times the amount of hemoglobin in the body tissue before contacting the angiogenic agent.

The amount of using or compounding the angiogenic agent is not particularly limited. For example, in a case of forming a transplant site that exhibits an excellent engraftment rate of a transplant material, it is preferably an amount that can induce angiogenesis in terms of the amount of hemoglobin of from 2 mg/g tissue to 50 mg/g tissue, more preferably from 3 mg/g tissue to 40 mg/g tissue, further preferably from 4 mg/g tissue to 30 mg/g tissue, yet further preferably from 5 mg/g tissue to 25 mg/g tissue.

The amount of compounding SEK-1005 or the angiogenic agent in the angiogenic device is not particularly limited. For example, it is preferably the amount with which the amount of hemoglobin can be increased to from 5 times to 150 times, more preferably from 10 times to 100 times, further preferably from 15 times to 70 times the amount of hemoglobin before implanting the angiogenic device in the subcutaneous tissue for 10 days.

The amount of compounding SEK-1005 or the angiogenic agent in the angiogenic agent depends on various factors and is not particularly limited. For example, it is preferably the amount with which the amount of hemoglobin can be increased to from twice to 15 times, more preferably from twice to 10 times, further preferably from twice to 5 times the amount of hemoglobin in the subcutaneous tissue in a case in which a device not containing SEK-1005 or the angiogenic agent is implanted in the subcutaneous tissue under the same conditions.

The amount of using or compounding the angiogenic agent is preferably, for example, approximately from 50 µg to 1 mg in a 4.5% agarose gel rod having a size of 25 mm in length and 4 mm in diameter.

The method of contacting the angiogenic agent with a body tissue is not particularly limited. Examples of the method include a method of implanting the angiogenic agent in a body tissue together with other material(s) and releasing the same, and a method of administrating the angiogenic agent by injection on a regular basis. From the viewpoint of securing a time for the angiogenic agent to contact a body tissue, the method of implanting the angiogenic agent in a body tissue together with other material(s) and releasing the same is preferred. This method is preferred also because a space for a transplant material to be transplanted is created after removing the angiogenic agent. The size (area) of a body tissue to be contacted with the angiogenic agent is not particularly limited, and may be selected according to the purpose or the method for treatment.

The number of times for contacting the angiogenic agent to a body tissue is not particularly limited, and a desired transplant site can be formed by a single contact or by multiple contacts. The time period for contacting the angiogenic agent with a body tissue is not particularly limited. Although it depends on the factors such as a position of the body tissue, the amount of the angiogenic agent or the method of contacting the angiogenic agent to the body tissue, generation of blood vessels is observed in a body tissue usually at approximately 5 to 10 days after the contact. In an embodiment, in which a subcutaneous tissue is contacted with the angiogenic agent, the time period for the amount of hemoglobin to become at least twice the amount of hemoglobin before the contact is from 5 days to 20 days.

The angiogenic agent may consist only of SEK-1005, or may include a component other than SEK-1005. Examples of the component include a diluent, a disintegrant, a binder, a lubricant, a surfactant, a solubilizer, a stabilizer, a tonicity agent, a suspending agent, an emulsifier, a buffer, and a solvent. When the angiogenic agent includes a component other than SEK-1005, the content of SEK-1005 is not particularly limited, as long as SEK-1005 exhibits its effect as an active ingredient of the angiogenic agent.

The body tissue in which angiogenesis has been induced with the angiogenic agent is suitable for transplanting a transplant material, and it is possible to improve the engraftment rate of a transplant material, for example.

With regard to the details and the specific embodiments of the body tissue in which angiogenesis has been induced with the angiogenic agent, in a case in which it is used as a transplant site, the details and the specific embodiments as described above concerning the transplant-forming agent can be referred to. With regard to the details and the specific embodiments of the transplant material, the details and the specific embodiments as described above concerning the transplant material can be referred to.

In an embodiment, the angiogenic agent is used for forming a transplant site in a subcutaneous tissue for transplanting pancreatic islets. In a subcutaneous tissue in which angiogenesis has been induced with the angiogenic agent, blood vessels having a small diameter are formed in an amount that is sufficient for engraftment of the transplanted pancreatic islets, and it is highly suitable for transplantation of pancreatic islets. Therefore, the pancreatic islets can secrete a sufficient amount of insulin for controlling the blood glucose level, and elevate the plasma insulin concentration of an individual after the transplantation to a degree higher than the plasma insulin concentration before the transplantation. As a result, the blood glucose level can be controlled to a degree lower than the blood glucose level before the transplantation. For example, it is possible to increase the plasma insulin concentration of an individual after the transplantation of pancreatic islets to at least twice, or at least three times, or at least four times the plasma insulin concentration of the individual before the transplantation. Further, for example, it is possible to control the blood glucose level of an individual after the transplantation of pancreatic islets to be close to a normal range, or to control the fluctuation in the blood glucose level to be close to a normal range. The "plasma insulin concentration" and the "blood glucose level" as mentioned herein are based on casual measurements, respectively.

In an embodiment, pancreatic islets that have been transplanted in a subcutaneous tissue, in which angiogenesis has been induced with the angiogenic agent, can secrete insulin that is sufficient for controlling the blood glucose level in a continuous manner. For example, it is possible to maintain a state in which the plasma insulin concentration of an individual after the transplantation of the pancreatic islets is higher than the plasma insulin concentration of the individual before the transplantation, or to maintain a state in which the blood glucose level of an individual after the transplantation is lower than the blood glucose level of the individual before the transplantation, for 10 days or more, 20 days or more, 30 days or more, 60 days or more, 100 days or more, 110 days or more, or 120 days or more.

In an embodiment, the transplant material, which has been transplanted to a transplant site formed with the angiogenic agent, is engrafted to the transplant site. The reason for this is considered to be that SEK-1005 included in the angiogenic agent functions to induce angiogenesis and to form a suitable transplant site. According to the angiogenic agent, it is possible to engraft a transplant material even if the donor and the recipient are different individuals.

### <Angiogenic device>

The angiogenic device includes SEK-1005 or a salt thereof, or the angiogenic agent of the embodiment and a substrate that retains SEK-1005 and the angiogenic agent. When a body tissue is contacted with the angiogenic device, angiogenesis is induced in the body tissue. More specifically, the angiogenic agent is released from the inside of the angiogenic device that is in contact with a body tissue, and acts on the body tissue to induce angiogenesis.

The body tissue in which angiogenesis has been induced by the angiogenic agent is suitable especially as a transplant site for a tissue such as a cell or an organ. The details of the angiogenic agent included in the angiogenic device are as described above. With regard to the details and the specific embodiments such as the type or the structure of the substrate included in the angiogenic device, and the method of using the angiogenic device, the descriptions regarding the transplant site-forming device can be referred to.

### <Use of transplant site-forming agent>

The use of the transplant site-forming agent, the transplant site-forming device, the angiogenic agent or the angiogenic device (hereinafter, also collectively referred to as the transplant site-forming device) is as defined in the claims. Examples thereof include a use for forming a transplant site, a use for inducing angiogenesis, a use for transplanting a transplant material, a use for allowing a transplant material to survive under a skin, a use for treating diabetes, a use for controlling a blood glucose level, and a use for controlling a plasma insulin concentration.

### <Method of forming transplant site or method of inducing angiogenesis>

The method of forming a transplant site disclosed herein includes a process of contacting, with a body tissue, the transplant site-forming agent or the transplant site-forming device. By this method, SEK-1005 included in the transplant site-forming agent or the transplant site-forming device is released to the body tissue, thereby forming a transplant site.

The method of inducing angiogenesis disclosed herein includes a process of contacting, with a body tissue, the angiogenic agent or the angiogenic device. By this method, SEK-1005 included in the angiogenic agent or the angiogenic device is released to the body tissue, thereby inducing angiogenesis.

The position for forming a transplant site or the position for inducing angiogenesis is not particularly limited. For example, the method of forming a transplant site or the method of inducing angiogenesis is suitable as a method of forming a transplant site or as a method of inducing angiogenesis in a body tissue in which the amount of blood vessels is relatively small, such as a subcutaneous tissue. In a case in which a transplant site is formed or angiogenesis is induced in a subcutaneous tissue, the position to which the transplant site-forming agent is to be contacted may be a surface of a subcutaneous tissue (an interface with a dermic layer) or may be inside a subcutaneous tissue.

The method of contacting the transplant site-forming agent is not particularly limited, and may be selected according to the position for forming the transplant site or inducing angiogenesis. In a case of a subcutaneous tissue, the transplant site-forming agent is implanted by an ordinary method and left for a period of time until a desired amount of blood vessels are formed. In order to induce angiogenesis efficiently, it is preferred to allow SEK-1005 to be released from the transplant site-forming agent that has been implanted. The time period for leaving the transplant site-forming agent after implanting the same is preferably from 1 day to 35 days, more preferably from 2 day to 28 days, further preferably from 3 days to 21 days, particularly preferably from 4 days to 14 days.

Once a desired transplant site is formed, or angiogenesis is induced, by contacting the transplant site-forming agent with a body tissue, the transplant site-forming agent may be removed from the body tissue or may not be removed. In a case in which the transplant site-forming agent is removed, a space created after the removal of the transplant site-forming agent may be utilized as a space for transplanting a transplant material. The shape or size of the transplant site is not particularly limited, and may be determined according to the type, or the amount of the transplant material.

The transplant material starts to function usually at approximately several days after the transplantation of the same to the transplant site formed with the transplant site-forming agent. It is possible to administer additional SEK-1005 by injection to the transplant site to which the transplant material has been transplanted. As necessary, other chemical(s) or an immune suppresser may be used in combination.

In the transplant site formed with the transplant site-forming agent, a state of engraftment is maintained even if a transplant material obtained from a different individual is transplanted thereto.

In the methods as mentioned above, with regard to the details and the specific embodiments of the transplant site, the details and the specific embodiments as described above regarding the transplant site-forming agent may be referred to. With regard to the details and the specific embodiments of the transplant material, the details and the specific embodiments of the transplant material as mentioned above may be referred to.

### <Method of transplanting transplant material or method of promoting engraftment of transplant material to transplant site>

The method of transplanting a transplant material or the method of promoting engraftment of a transplant material to a transplant site disclosed herein includes: a process of forming a transplant site by contacting the transplant site-forming agent with a body tissue; and a process of transplanting a transplant material to the transplant site.

The process of transplanting a transplant material to the transplant site may include a process of transplanting a transplant material selected from the group consisting of a cell of the same kind, a microtissue of the same kind, a cell of a different kind, and a microtissue of a different kind. According to the method, a transplant site that exhibits an excellent engraftment rate of a transplant material can be formed at a desired portion of a body. Further, according to the method, a state of engraftment is maintained even if the transplant material is obtained from a different individual.

The process of forming a transplant site may include a process of inducing angiogenesis that is suitable for forming a transplant site, by contacting an angiogenic agent or an angiogenic device, the agent or the device containing SEK-1005.

In an embodiment of the method, the transplant material is a transplant material that secretes a physiologically active substance. In an embodiment of the method, the transplant material that secretes a physiologically active substance is a pancreatic islet. In an embodiment of the method, the transplant site is a subcutaneous tissue. In the method, with regard to the details and the specific embodiments of the transplant site, the details and the specific embodiments as described above regarding the transplant site-forming agent may be referred to. With regard to the details and the specific embodiments of the transplant material, the details and the specific embodiments of the transplant material as mentioned above may be referred to.

### <Method of treating diabetes, method of controlling blood glucose level and method of controlling plasma insulin concentration>

The method of treating diabetes, the method of controlling a blood glucose level, or the method of controlling a plasma insulin concentration disclosed herein includes: a process of forming a transplant site by contacting the transplant site-forming agent with a body tissue; and a process of transplanting a pancreatic islet to the transplant site. According to the method, it is possible to form a transplant site that exhibits an excellent engraftment rate of a pancreatic islet at a desired position of a body. Further, according to the method, a state of engraftment is maintained even if the pancreatic islet is obtained from a different individual.

The process of forming a transplant site may include a process of inducing angiogenesis that is suitable for forming a transplant site, by contacting an angiogenic agent or an angiogenic device, the agent or the device containing SEK-1005.

The diabetes to be treated by the method is not particularly limited, as long as the method is effective for the treatment, and includes insulin-dependent diabetes (type 1 diabetes), non-insulin-dependent diabetes (type 2 diabetes) and various complications relevant to diabetes.

In the method, with regard to the details and the specific embodiments of the transplant site, the details and the specific embodiments as described above regarding the transplant site-forming agent may be referred to. With regard to the details and the specific embodiments of the transplant material, the details and the specific embodiments of the transplant material as mentioned above may be referred to.

### Examples

In the following, the invention will be explained in further detail with reference to the examples. The material, amount, proportion, or procedure may be modified, as long as it does not deviate from the gist of the invention as defined in the claims. Accordingly, the scope of the invention should not be construed in a limited way as the specific examples described below.

### <Example 1 Evaluation of effect of inducing angiogenesis>

Diabetic rats were prepared by intraperionerally administering streptozocin (STZ) to ACI rats (8-week old, male). The blood glucose level of the rats were measured, and it was determined that diabetes was induced when the blood glucose level exceeded 400 mg/dL for 2 days in succession. A substrate formed of 4.5% agarose gel having a rod shape of 25 mm in length and 4 mm in diameter was impregnated with SEK-1005 (100 µg) dissolved in alcohol. After sufficiently evaporating alcohol, the agarose rod was impregnated with physiological saline. The agarose rods were implanted under the skin of the diabetic rats at two portions, on the right side and on the left side of the backbone, respectively. As a control, agarose rods that were impregnated with physiological saline without SEK-1005 were implanted under the skin of a diabetic rat, on the right side and on the left side of the backbone, respectively.

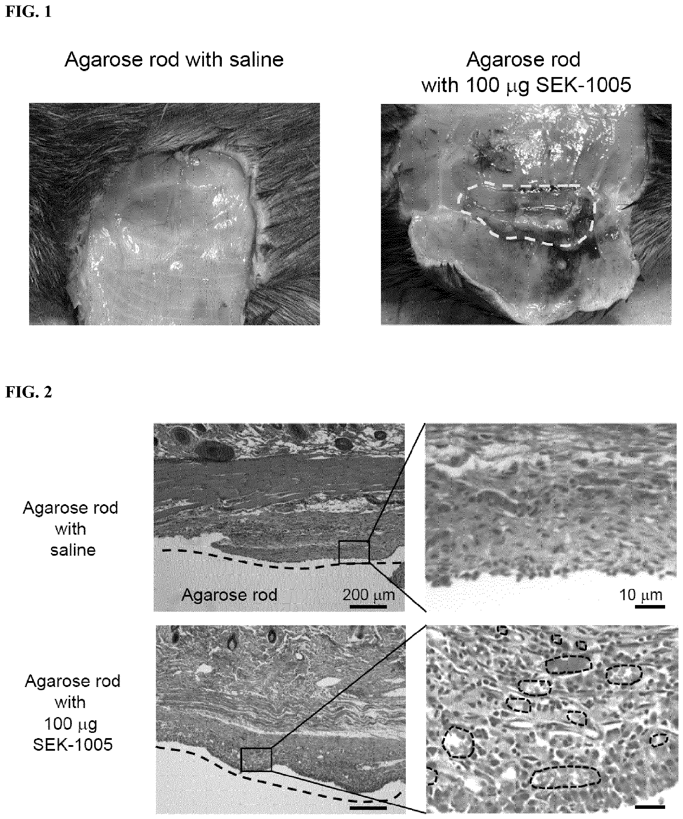

Ten days after the implantation, the agarose rods were removed and the surrounding tissues were observed. The result is shown in Fig. 1. As shown in Fig. 1, blood vessels having a small diameter were developed at the surrounding tissue at which the agarose rod containing SEK-1005 had been implanted (indicated by a dashed line), as compared to the surrounding tissue at which the agarose rod not containing SEK-1005 had been implanted.

Fig. 2 shows a slice of a hematoxylin-eosin (HE)-stained subcutaneous tissue at a contact portion with the agarose rod. As shown in Fig. 2, blood vessels having a small diameter were developed in the subcutaneous tissue that had been contacted with the agarose rod containing SEK-1005 (indicated by a dashed line), as compared with the subcutaneous tissue that had been contacted with the agarose rod not containing SEK-1005.

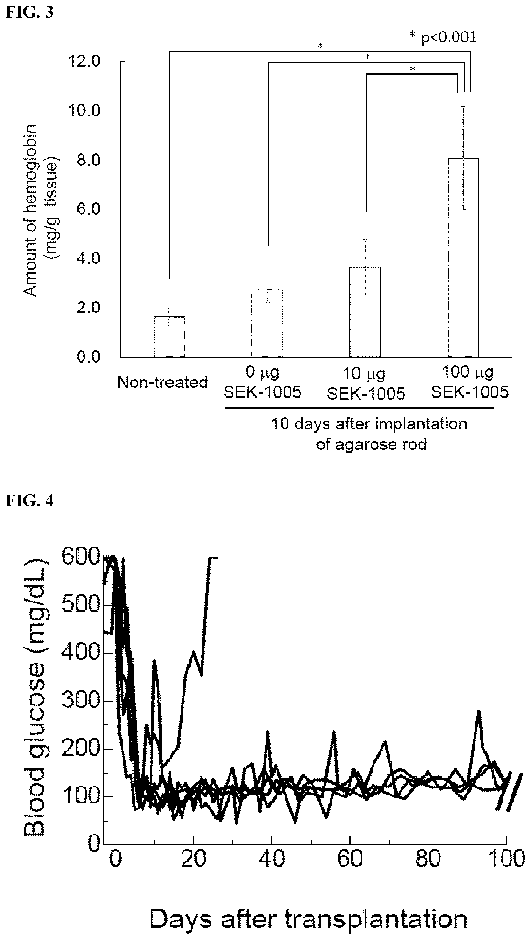

Fig. 3 shows the results of measuring the amount of hemoglobin in a subcutaneous tissue of a contact portion with the agarose rod. The data shown in Fig. 3 are the measurement results of the amount of hemoglobin of a diabetic ACI rat to which an agarose rod was not implanted, a diabetic rat implanted with an agarose rod not containing SEK-1005, a diabetic rat implanted with an agarose rod containing 10 µg of SEK-1005, and a diabetic rat implanted with an agarose rod containing 100 µg of SEK-1005. The measurement was conducted after removing the agarose rod that had been implanted for 10 days.

As shown in Fig. 3, it was observed that the amount of hemoglobin in the subcutaneous tissue was increased upon contact with SEK-1005. The error bars in Fig. 3 each indicate standard deviations (non-treated; n=3, 0 µg SEK-1005; n=3, 10 µg SEK-1005; n=3, 100 µg SEK-1005; n=5).

### <Example 2 Evaluation of effect of controlling blood glucose level>

Pancreatic islets were separated from a F344 rat as a donor, and were transplanted to the diabetic ACI rats as prepared above as recipients. Specifically, the agarose rods containing SEK-1005 as prepared in the same process as described above were implanted on the right side and on the left side of the backbone of the diabetic rats, respectively, and were removed 10 days after the implantation. Thereafter, 1500 pancreatic islets separated from the F433 rat were transplanted to each of the spaces formed by removing the agarose rods, respectively (3000 pancreatic islets in total).

As a control, agarose rods not containing SEK-1005 were implanted on the right side and on the left side of the backbone of the diabetic rats, respectively, and were removed 10 days after the implantation. Thereafter, 1500 pancreatic islets separated from the F433 rat were transplanted to each of the spaces formed by removing the agarose rods, respectively (3000 pancreatic islets in total).

Fig. 4 shows the blood glucose levels of the recipients after removing the agarose rods containing SEK-1005 and transplanting the pancreatic islets. The measurement was contacted every day for 2 weeks after the transplantation, and once in 1 to 3 days thereafter. As shown in Fig. 4, the blood glucose levels of four in six recipients were normalized over 100 days. One of the recipients died in an accident after 60 days, but its blood glucose level was normalized. The measurement was conducted on a casual blood glucose level.

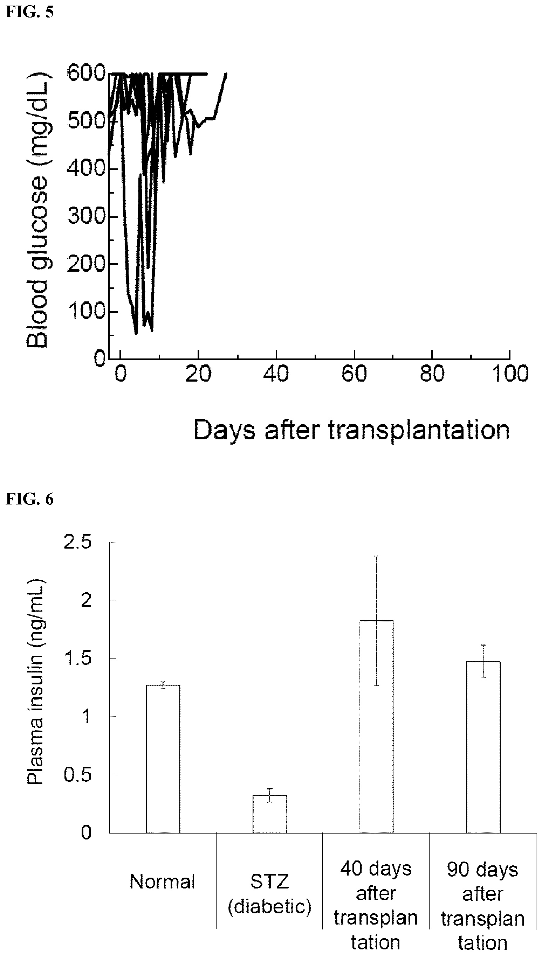

Fig. 5 shows the blood glucose levels of the recipients after removing the agarose rods not containing SEK-1005 and transplanting the pancreatic islets. The measurement was conducted every day for 2 weeks after the transplantation, and once in 2 to 3 days thereafter. As shown in Fig. 5, the blood glucose levels of four in five recipients were over 400 mg/dL and never reached a normal level. The blood glucose level of one of the five recipients reached a normal level temporarily, but again became over 400 mg/dL approximately 10 days thereafter.

Fig. 6 shows the measurement results of the plasma insulin concentration of the recipients after transplanting the pancreatic islets. As shown in Fig. 6, the plasma insulin concentration of the recipients, at 40 days and at 90 days after removing the agarose rods containing SEK-1005 and transplanting the pancreatic islets, was significantly higher than the plasma insulin concentration of a diabetic rat to which pancreatic islets were not transplanted.

Moreover, the plasma insulin concentration of the recipients at 40 days and at 90 days after transplanting the pancreatic islets did not show a significant difference with respect to the plasma insulin concentration of a normal rat, and similar levels were maintained. The error bars in Fig. 6 each indicate standard deviations (normal; n=4, diabetic; n=3, 40 days after transplantation; n=4, 90 days after transplantation; n=4). The values of the plasma insulin concentration are casual measurements.

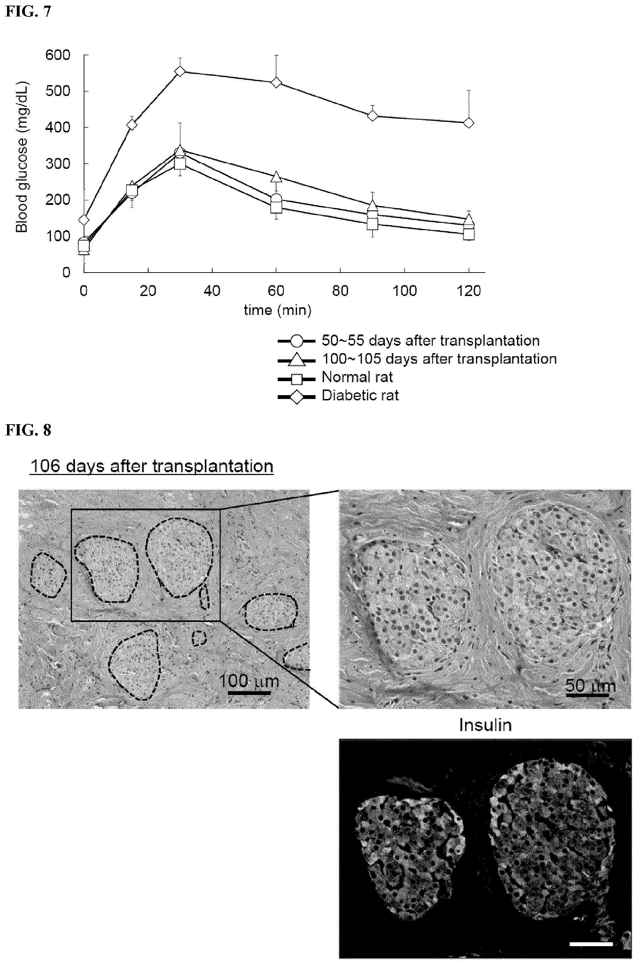

Fig. 7 shows the results of a glucose tolerance test conducted on the recipients at 50 to 55 days after or at 100 to 105 days after the transplantation of the pancreatic islets. In the test, changes in the blood glucose level after intraperitoneally administering glucose (60 mg/kg) were examined. As shown in Fig. 7, changes in the blood glucose level of the recipients at 50 to 55 days after the transplantation of the pancreatic islets were controlled to a similar level to a normal rat.

Further, glucose was administered in the same manner as the above to the recipients at 100 to 105 days after the transplantation of pancreatic islets, and the changes in the blood glucose level were examined. As a result, a trend that a degree of decrease in blood glucose level was less than the recipients at 50 to 55 days after the transplantation of the pancreatic islets was observed. Nonetheless, the results show, as a whole, that the blood glucose level was controlled to a degree similar to that of a normal rat. On the other hand, the diabetic rats maintained a blood glucose level of as high as approximately 500 mg/dL. The error bars in Fig. 7 each indicate standard deviations (normal; n=4, diabetic; n=3, 40 days after transplantation; n=4, 90 days after transplantation; n=4).

The upper images in Fig. 8 are photographs of a HE-stained tissue slice prepared from a site at which the pancreatic islets were transplanted, obtained from a recipient with a normalized blood glucose level at 106 days after the transplantation of the pancreatic islets. As shown in the images, many cell clumps which were considered to be pancreatic islets existed (indicated by dashed line) and many blood vessels having a small diameter penetrated the cell clumps.

The lower image in Fig. 8 is an immunofluorescent-stained image of a tissue slice obtained at the substantially same site to the HE-stained tissue slice. As a result of observation with a fluorescence microscope, insulin-positive cells were observed at positions corresponding to the cell clumps, considered to be pancreatic islets, were observed in the HE-stained image.

The results as shown above show that the invention enables transplantation of a transplant material with an excellent engraftment rate.

## Claims

1. SEK-1005 (Ser, 3-hydroxy-N-[2-hydroxy-1-oxo-2-tetrahydro-2-hydroxy-6-methyl-5-(2-methylpropyl)-2H-pyran-2-yl-propyl]-Leu-Pip(hexahydro-3-pyridazinecarbonyl)-N-hydroxy-Ala-N-methyl-Phe-Pip-rho-lactone) or a salt thereof for use in a method of forming a transplant site which is suitable for transplantation of a transplant material, said method comprising contacting SEK-1005 or a salt thereof with a body tissue in a living body.

2. The SEK-1005 or salt thereof for use according to claim 1, wherein the transplant site is formed in a subcutaneous tissue.

3. The SEK-1005 or salt thereof for use according to claim 1 or claim 2, wherein a transplant material is transplanted to the transplant site, the transplant material being selected from the group consisting of a cell, a tissue, a cell that is formed by differentiation induction of an embryonic stem cell or an induced pluripotent stem cell, and a tissue that is formed by differentiation induction of an embryonic stem cell or an induced pluripotent stem cell.

4. The SEK-1005 or salt thereof for use according to any one of claims 1 to 3, wherein a transplant material that secretes a physiologically active substance is transplanted to the transplant site.

5. The SEK-1005 or salt thereof for use according to claim 4, wherein the transplant material that secretes a physiologically active substance is selected from the group consisting of a pancreatic islet, a hepatic cell, an adrenal gland, a parathyroid, a cell that produces erythropoietin, a cell that produces growth hormone, a transplant material produced from a somatic stem cell, a transplant material produced from an induced pluripotent stem cell, and a transplant material produced from an embryonic stem cell.

6. The SEK-1005 or salt thereof for use according to claim 5, wherein the transplant material that secretes a physiologically active substance is a pancreatic islet, preferably wherein a plasma insulin concentration of an individual after transplantation of the pancreatic islet becomes higher than a plasma insulin concentration of the individual before the transplantation, more preferably wherein the plasma insulin concentration of the individual after transplantation of the pancreatic islet is at least twice the plasma insulin concentration of the individual before the transplantation.

7. The SEK-1005 or salt thereof for use according to claim 6, wherein a state in which the plasma insulin concentration of the individual after transplantation of the pancreatic islet is higher than the plasma insulin concentration of the individual before the transplantation continues for 10 days or more.

8. The SEK-1005 or salt thereof for use according to claim 6 or claim 7, wherein the blood glucose level of the individual after transplantation of the pancreatic islet becomes lower than the blood glucose level of the individual before the transplantation, preferably wherein a state in which the blood glucose level of the individual after transplantation of the pancreatic islet is lower than the blood glucose level of the individual before the transplantation continues for 10 days or more.

9. The SEK-1005 or salt thereof for use according to any one of claims 1 to 8, wherein the transplant site is formed by inducing angiogenesis in a subcutaneous tissue.

10. A transplant site-forming device that comprises the SEK-1005 or salt thereof according to claim 1 and a substrate that retains the SEK-1005 or salt thereof, preferably wherein the substrate is formed from a biocompatible material for use in forming a transplant site by implanting the transplant site-forming device under the skin of an individual and allowing SEK-1005 or a salt thereof to be released to a tissue surrounding the transplant site-forming device.

11. The transplant site-forming device according to claim 10, wherein the substrate is formed from at least one selected from the group consisting of a hydrogel, a dried hydrogel, a sponge, a porous polymer block, a porous body, a porous sheet and a porous film, preferably wherein the substrate is formed from a biocompatible material.

12. The transplant site-forming device for use according to claim 10 or claim 11 that is removed or not removed after formation of the transplant site.

13. SEK-1005 or a salt thereof as defined in claim 1 for use in induction of angiogenesis.

14. The SEK-1005 or salt thereof for use according to claim 13, wherein the angiogenesis is suitable for forming a transplant site, transplanting a transplant material, promoting engraftment of a transplant material at a transplant site, treating diabetes, controlling a blood glucose level, or controlling a plasma insulin concentration.

15. An angiogenic device that comprises the SEK-1005 or salt thereof as defined in claim 1 and a substrate that retains the SEK-1005 or salt thereof for use in induction of angiogenesis.

## Patentansprüche

1. SEK-1005 (Ser, 3-hydroxy-N-[2-hydroxy-1-oxo-2-tetrahydro-2-hydroxy-6-methyl-5-(2-methylpropyl)-2H-pyran-2-yl-propyl]-Leu-Pip(hexahydro-3-pyridazinecarbonyl)-N-hydroxy-Ala-N-methyl-Phe-Pip-rho-Lacton) oder ein Salz davon zur Verwendung in einem Verfahren zum Bilden eines Transplantationsortes, der für eine Transplantation eines Transplantationsmaterials geeignet ist, wobei das Verfahren Inkontaktbringen von SEK-1005 oder eines Salzes davon mit einem Körpergewebe in einem lebendigen Körper umfasst.

2. SEK-1005 oder ein Salz davon zur Verwendung nach Anspruch 1, wobei der Transplantationsort in einem subkutanen Gewebe gebildet wird.

3. SEK-1005 oder ein Salz davon zur Verwendung nach Anspruch 1 oder Anspruch 2, wobei ein Transplantationsmaterial an den Transplantationsort transplantiert wird, wobei das Transplantationsmaterial ausgewählt wird aus der Gruppe bestehend aus einer Zelle, einem Gewebe, einer Zelle, die durch Differenzierungsinduktion einer embryonalen Stammzelle oder einer induzierten pluripotenten Stammzelle gebildet wird, und einem Gewebe, das durch Differenzierungsinduktion einer embryonalen Stammzelle oder einer pluripotenten Stammzelle gebildet wird.

4. SEK-1005 oder ein Salz davon zur Verwendung nach einem der Ansprüche 1 bis 3, wobei ein Transplantationsmaterial, das eine physiologisch aktive Substanz absondert, an den Transplantationsort transplantiert wird.

5. SEK-1005 oder ein Salz davon zur Verwendung nach Anspruch 4, wobei das Transplantationsmaterial, das eine physiologisch aktive Substanz absondert, ausgewählt wird aus der Gruppe bestehend aus einer Inselzelle der Bauchspeicheldrüse, einer hepatischen Zelle, einer Nebenniere, einer Nebenschilddrüse, einer Zelle, die Erythropoetin erzeugt, einer Zelle, die Wachstumshormone erzeugt, einem Transplantationsmaterial, das aus einer somatischen Stammzelle erzeugt wird, einem Transplantationsmaterial, das aus einer induzierten pluripotenten Stammzelle erzeugt wird, und einem Transplantationsmaterial, das aus einer embryonalen Stammzelle erzeugt wird.

6. SEK-1005 oder ein Salz davon zur Verwendung nach Anspruch 5, wobei das Transplantationsmaterial, das eine physiologisch aktive Substanz absondert, eine Inselzelle der Bauchspeicheldrüse ist, wobei vorzugsweise eine Plasmainsulinkonzentration einer Person nach Transplantation der Inselzelle der Bauchspeicheldrüse höher wird als eine Plasmainsulinkonzentration der Person vor der Transplantation, wobei die Plasmainsulinkonzentration der Person noch bevorzugter nach der Transplantation der Inselzelle der Bauchspeicheldrüse mindestens zweimal so hoch wie die Plasmainsulinkonzentration der Person vor der Transplantation ist.

7. SEK-1005 oder ein Salz davon zur Verwendung nach Anspruch 6, wobei sich ein Zustand, in dem die Plasmainsulinkonzentration der Person nach Transplantation der Inselzelle der Bauchspeicheldrüse höher ist als eine Plasmainsulinkonzentration der Person vor der Transplantation, 10 Tage lang oder mehr fortsetzt.

8. SEK-1005 oder ein Salz davon zur Verwendung nach Anspruch 6 oder Anspruch 7, wobei der Blutglukosewert der Person nach Transplantation der Inselzelle der Bauchspeicheldrüse niedriger wird als der Blutglukosewert der Person vor der Transplantation, wobei sich vorzugsweise ein Zustand, in dem der Blutglukosewert der Person nach Transplantation der Inselzelle der Bauchspeicheldrüse niedriger als der der Blutglukosewert der Person vor der Transplantation ist, 10 Tage lang oder mehr fortsetzt.

9. SEK-1005 oder ein Salz davon zur Verwendung nach einem der Ansprüche 1 bis 8, wobei der Transplantationsort durch Induzieren einer Angiogenese in einem subkutanen Gewebe gebildet wird.

10. Transplantationsortbildungsvorrichtung, die SEK-1005 oder ein Salz davon nach Anspruch 1 und ein Substrat, das SEK-1005 oder ein Salz davon zurückhält, umfasst, wobei das Substrat vorzugsweise aus einem biokompatiblen Material zur Verwendung beim Bilden eines Transplantationsortes durch Implantieren der Transplantationsortbildungsvorrichtung unter die Haut einer Person gebildet wird, und es SEK-1005 oder einem Salz davon erlaubt, in ein Gewebe freigesetzt zu werden, das die Transplantationsortbildungsvorrichtung umgibt.

11. Transplantationsortbildungsvorrichtung nach Anspruch 10, wobei das Substrat aus mindestens einem gebildet wird, ausgewählt aus der Gruppe bestehend aus einem Hydrogel, einem getrockneten Hydrogel, einem Schwamm, einem porösen Polymerblock, einem porösen Körper, einer porösen Folie und einem porösen Film, wobei das Substrat vorzugsweise aus einem biokompatiblen Material gebildet wird.

12. Transplantationsortbildungsvorrichtung zur Verwendung nach Anspruch 10 oder Anspruch 11, die nach Bildung des Transplantationsortes entfernt wird oder nicht.

13. SEK-1005 oder ein Salz davon nach Anspruch 1, zur Verwendung bei einer Induktion einer Angiogenese.

14. SEK-1005 oder ein Salz davon nach Anspruch 13, wobei sich die Angiogenese zur Bildung eines Transplantationsortes, zum Transplantieren eines Transplantationsmaterials, zur Unterstützung der Verpflanzung eines Transplantationsmaterials an einem Transplantationsort, zur Behandlung von Diabetes, zur Steuerung eines Blutglukosewertes, oder Steuerung einer Plasmainsulinkonzentration eignet.

15. Angiogene Vorrichtung, die SEK-1005 oder ein Salz davon nach Anspruch 1 und ein Substrat umfasst, das SEK-1005 oder ein Salz davon zur Verwendung bei Induktion einer Angiogenese zurückhält.

## Revendications

1. SEK-1005 (Ser, 3-hydroxy-N-[2-hydroxy-1-oxo-2-tétrahydro-2-hydroxy-6-méthyl-5-(2-méthylpropyl)-2H-pyran-2-yl-propyl]-Leu-Pip(hexahydro-3-pyridazinecarbonyl)-N-hydroxy-Ala-N-méthyl-Phe-Pip-rho-lactone) ou un sel de celle-ci pour utilisation dans un procédé de formation d'un site de transplantation qui est approprié pour la transplantation d'un matériau de transplantation, ledit procédé comprenant la mise en contact de SEK-1005 ou d'un sel de celle-ci avec un tissu corporel dans un corps vivant.

2. SEK-1005 ou sel de celle-ci pour utilisation selon la revendication 1, dans laquelle ou lequel le site de transplantation est formé dans un tissu sous-cutané.

3. SEK-1005 ou sel de celle-ci pour utilisation selon la revendication 1 ou la revendication 2, dans laquelle ou lequel un matériau de transplantation est transplanté dans le site de transplantation, le matériau de transplantation étant choisi dans le groupe consistant en une cellule, un tissu, une cellule qui est formée par une induction de différentiation cellulaire d'une cellule souche embryonnaire ou d'une cellule souche pluripotente induite, et un tissu qui est formé par une induction de différentiation cellulaire d'une cellule souche embryonnaire ou d'une cellule souche pluripotente induite.

4. SEK-1005 ou sel de celle-ci pour utilisation selon l'une quelconque des revendications 1 à 3, dans laquelle ou lequel un matériau de transplantation qui secrète une substance physiologiquement active est transplanté dans le site de transplantation.

5. SEK-1005 ou sel de celle-ci pour utilisation selon la revendication 4, dans laquelle ou lequel le matériau de transplantation qui secrète une substance physiologiquement active est choisi dans le groupe consistant en un îlot pancréatique, une cellule hépatique, une glande surrénale, une parathyroïde, une cellule qui produit de l'érythropoïétine, une cellule qui produit de l'hormone de croissance, un matériau de transplantation produit à partir d'une cellule souche somatique, un matériau de transplantation produit à partir d'une cellule souche pluripotente induite, et un matériau de transplantation produit à partir d'une cellule souche embryonnaire.

6. SEK-1005 ou sel de celle-ci pour utilisation selon la revendication 5, dans laquelle ou lequel le matériau de transplantation qui secrète une substance physiologiquement active est un îlot pancréatique, dans lequel de préférence une concentration en insuline plasmatique d'un individu après transplantation de l'îlot pancréatique devient plus élevée qu'une concentration en insuline plasmatique de l'individu avant la transplantation, dans lequel de manière davantage préférée la concentration en insuline plasmatique de l'individu après transplantation de l'îlot pancréatique est au moins le double de la concentration en insuline plasmatique de l'individu avant la transplantation.

7. SEK-1005 ou sel de celle-ci pour utilisation selon la revendication 6, dans laquelle ou lequel un état dans lequel la concentration en insuline plasmatique de l'individu après transplantation de l'îlot pancréatique est plus élevée que la concentration en insuline plasmatique de l'individu avant la transplantation se poursuit pendant 10 jours ou plus.

8. SEK-1005 ou sel de celle-ci pour utilisation selon la revendication 6 ou la revendication 7, dans laquelle ou lequel le taux de glycémie de l'individu après transplantation de l'îlot pancréatique devient inférieur au taux de glycémie de l'individu avant la transplantation, dans lequel de préférence un état dans lequel le taux de glycémie de l'individu après transplantation de l'îlot pancréatique est inférieur au taux de glycémie de l'individu avant la transplantation se poursuit pendant 10 jours ou plus.

9. SEK-1005 ou sel de celle-ci pour utilisation selon l'une quelconque des revendications 1 à 8, dans laquelle ou lequel le site de transplantation est formé en induisant une angiogenèse dans un tissu sous-cutané.

10. Dispositif de formation de site de transplantation qui comprend la SEK-1005 ou un sel de celle-ci selon la revendication 1 et un substrat qui retient la SEK-1005 ou un sel de celle-ci, dans lequel de préférence le substrat est formé à partir d'un matériau biocompatible pour utilisation dans la formation d'un site de transplantation en implantant le dispositif de formation de site de transplantation sous la peau d'un individu et en laissant se libérer la SEK-1005 ou un sel de celle-ci dans un tissu entourant le dispositif de formation de site de transplantation.

11. Dispositif de formation de site de transplantation selon la revendication 10, dans lequel le substrat est formé à partir d'au moins un élément choisi dans le groupe consistant en un hydrogel, un hydrogel séché, une éponge, un bloc de polymère poreux, un corps poreux, une feuille poreuse et un film poreux, dans lequel de préférence le substrat est formé à partir d'un matériau biocompatible.

12. Dispositif de formation de site de transplantation pour utilisation selon la revendication 10 ou la revendication 11 qui est éliminé ou non éliminé après formation du site de transplantation.

13. SEK-1005 ou sel de celle-ci selon la revendication 1 pour utilisation pour induire une angiogenèse.

14. SEK-1005 ou sel de celle-ci pour utilisation selon la revendication 13, dans laquelle ou lequel l'angiogenèse est appropriée pour former un site de transplantation, transplanter un matériau de transplantation, favoriser la prise de greffe d'un matériau de transplantation au niveau d'un site de transplantation, traiter le diabète, réguler un taux de glycémie, ou réguler une concentration en insuline plasmatique.

15. Dispositif angiogénique qui comprend la SEK-1005 ou un sel de celle-ci selon la revendication 1 et un substrat qui retient la SEK-1005 ou un sel de celle-ci pour utilisation pour induire une angiogenèse.
